**Europäisches Patentamt**

**European Patent Office**

**Office européen des brevets**

(19)

(11) Veröffentlichungsnummer : **0 142 812**
**B1**

(12)                    **EUROPÄISCHE PATENTSCHRIFT**

(45) Veröffentlichungstag der Patentschrift :
02.03.88

(21) Anmeldenummer : 84113664.1

(22) Anmeldetag : 13.11.84

(51) Int. Cl.⁴ : **C 07 C 33/02**, C 07 C 29/14

(54) 2-Ethyl-2-prenyl-3-hexenol, seine Herstellung und Verwendung als Riechstoff.

(30) Priorität : 17.11.83 DE 3341604

(43) Veröffentlichungstag der Anmeldung :
29.05.85 Patentblatt 85/22

(45) Bekanntmachung des Hinweises auf die Patenterteilung : 02.03.88 Patentblatt 88/09

(84) Benannte Vertragsstaaten :
CH DE FR GB LI NL

(56) Entgegenhaltungen :
US-A- 4 010 207
HOUBEN-WEYL "Methoden der Organischen Chemie", Band VI/1b (Alkohole III), 4. Auflage, Seiten
304, 310, 311, Georg Thieme Verlag, Stuttgart, New
York

(73) Patentinhaber : **Consortium für elektrochemische**
**Industrie GmbH**
**Zielstattstrasse 20**
**D-8000 München 70 (DE)**

(72) Erfinder : **Gebauer, Helmut, Dr. Dipl.-Chem.**
**Schaffhauser Strasse 18**
**D-8000 München 71 (DE)**
Erfinder : **Mehlin, Hans**
**Ammerseestrasse 8**
**D-8027 Neuried (DE)**

**Beschreibung**

Es ist gemäß US-PS-4010 207 2-Ethyl-2-prenyl-3-hexenal als Riechstoff bekannt geworden. Sein Duft wird als grün und süß beschrieben mit Noten von Tomate, Citrus und Rose.

Es wurde nun mit 2-Ethyl-2-prenyl-3-hexenol ein Riechstoff aufgefunden, dessen Geruchsbild überraschenderweise durch herbe und holzige Noten bestimmt wird und sich in Duftstoffkompositionen einpaßt, die typischerweise in der Herrenkosmetik verwendet werden. Der Duftstoff steht ferner aufgrund seiner Stabilitat sowohl in sauren als auch in alkalischen Medien zur Parfümierung einer wesentlich breiteren Produktpalette zur Verfügung, als sie bei Verwendung der chemisch labileren Aldehyde in Betracht käme.

Gegenstand der Erfindung ist 2-Ethyl-2-prenyl-3-hexenol.

Die Herstellung der erfindungsgemäßen Verbindung erfolgt durch Reduktion von 2-Ethyl-2-prenyl-3-hexenal. Beispiele für Reduktionsmittel sind komplexe Hydride wie Natriumboranat, Lithiumalanat, sowie Aluminiumisopropylat (Reduktion nach Meerwein/Ponndorf).

Ein bevorzugtes Verfahren zur Herstellung von 2-Ethyl-2-prenyl-3-hexenol ist dadurch gekennzeichnet, daß 2-Ethyl-2-prenyl-3-hexenal mit Aluminiumisopropylat umgesetzt wird.

Die Menge an Aluminiumisopropylat beträgt 0,05 - 1 Mol pro Mol umzusetzenden Aldehyds. Zweckmäßigerweise wird so vorgegangen, daß einer Lösung von Aluminiumisopropylat in Isopropanol der Aldehyd zudosiert wird, wobei Reaktionstemperaturen eingehalten werden, bei der als Nebenprodukt entstehendes Aceton (bzw. ein iso-Propanol/Aceton-Gemisch) abdestilliert.

Der als Ausgangsverbindung einzusetzende Aldehyd wird vorteilhafterweise im Eintopfverfahren durch Umsetzen von Butyraldehyd und Prenylchlorid in einem organisch/alkalischen 2-Phasensystem in Gegenwart eines Phasentransferkatalysators gewonnen. Unter diesen Reaktionbedingungen erfolgt die Bildung des Aldolkondensationsprodukts des Butyraldehyds unter gleichzeitiger Prenylierung des Produkts in $\alpha$-Stellung zur Aldehydgruppe.

Es werden etwa 2 Mol Butyraldehyd pro Mol Prenylchlorid eingesetzt. Das organisch/alkalische 2-Phasensystem wird gebildet aus einem organischen, mit Wasser nicht mischbaren, inerten Losungsmittel und einer 5-50 %igen, wässrigen Lösung oder in fester Form vorliegendem Alkalimetallhydroxid.

Als Phasentransferkatalysatoren werden biespielsweise Kronenether, quartare Ammonium- und Phosphoniumsalze eingesetzt in Mengen von 0,5 - 5 Mol %, bezogen auf Prenylchlorid.

Die Reaktionstemperaturen betragen 20-150°C, insbesondere 60-70°C.

Zweckmäßigerweise wird das Verfahren so durchgeführt, daß das 2-Phasensystem mit dem Katalysator vorgelegt und ein Gemisch der Reaktionkomponenten zugetropft wird.

Der vorstehend beschriebene Syntheseweg stellt das besonders bevorzugte Verfahren zur Herstellung von 2-Ethyl-2-prenyl-3-hexenol dar, das dadurch gekennzeichnet ist, daß

a) Butyraldehyd und Prenylchlorid im Eintopfverfahren in einem organisch/alkalischen 2-Phasensystem in Gegenwart eines Phasentransfer-Katalysators umgesetzt werden und

b) der gemäß a) erhaltene Aldehyd in Gegenwart von Aluminium-isopropylat reduziert wird.

Mit 2-Ethyl-2-prenyl-3-hexenol steht ein aus Basischemikalien vollsynthetisch zugänglicher Duftstoff zur Verfügung, dessen intensiver Geruch als grün-holzig, ambriert mit herb-würzigem Charakter beschrieben werden kann, der deutlich an Vetiver erinnert. Der Duftstoff verbindet sich vorteilhaft mit Patschouli, Eichen-moos-Produkten, Lavendel, Ionon, Opoponax u.a.

Der erfindungsgemäße Duftstoff wird zur Parfümierung kosmetischer und technischer Produkte eingesetzt. Sein Geruchsbild fügt sich insbesondere in Herrennoten ein, und unterstreicht die holzigen und balsamischen Nebennoten.

Die Erfindung wird nun an Hand von Beispielen näher erläutert :

Im folgenden sollen Zahlenangaben, die sich auf die Zusammensetzung von Duftstoffkompositionen beziehen, als Gewichtsteile verstanden werden.

Beispiel 1

Herstellung von 2-Ethyl-2-prenyl-3-henexol

In einem 2l-Vierhalskolben, versehen mit Rührer, Kolonne, Tropftrichter und Thermometer wurden 0,5 Mol Aluminiumiso-propylat und 1 Liter trockenen Iso-propanols vorgelegt. Der siedenden Mischung wurde eine Lösung von 1 Mol 2-Ethyl-2-prenyl-3-hexenal, gelöst in 200 ml trockenen Iso-propanols zugetropft. Das Reaktionsgemisch wurde standig soweit am Sieden erhalten, daß das entstehende Aceton über der Kolonne abgezogen werden konnte. Nachdem in Destillat kein Aceton mehr nachweisbar war, wurde das Isopropanol abdestilliert. Schließlich wurde der Rückstand in 250 ml 20 %ger Salzsäure eingetragen, die Phasen getrennt, die organische Phase mit Calciumchlorid getrocknet. Das Zielprodukt wurde noch über eine Vigreux-Kolonne destilliert. Siedepunkt 95°C bei 0,8 mbar. Die Ausbeute betrug 280 g entsprechend 95 % der Theorie.

# 0 142 812

Beispiel 2

« Holz-Base »

|  | a | b |
|---|---|---|
| Cypressenöl | 60 | 60 |
| Verdyl acetat (Givaudan Corp.) | 160 | 160 |
| Sandelholzöl | 140 | 140 |
| Bornylacetat | 40 | 40 |
| Benzylacetat | 20 | 20 |
| Baum-Moos farblos abs. | 50 | 50 |
| Patschouliöl | 50 | 50 |
| Linalylacetat | 80 | 80 |
| Eugenol | 30 | 30 |
| Labdanumöl (französisch) | 20 | 20 |
| Citronellol | 30 | 30 |
| Bergamottöl | 20 | 20 |
| Linalool | 110 | 110 |
| 6-Methyl-alpha-Ionon | 60 | 60 |
| 2-Ethyl-2-prenyl-3-hexenol | – | 130 |
|  | 870 | 1000 |

Die Komposition a besitzt einen weichen Duft von Edelhölzern. Durch Zugabe von 130 Gewichsteilen des erfindungsgemäßen Riechstoffs wird der Duft der Komposition a wärmer und runder. Die balsamische Note vom Baumharz wird betont, der neuhinzukommende Ambra-Charakter verleiht der Base einen wertvollen Duft.

Beispiel 3

Lavendelöl, künstlich

|  | a | b |
|---|---|---|
| Lavandinöl | 450 | 450 |
| Lavendelöl Barréme | 100 | 100 |
| Spikeöl span. | 100 | 100 |
| Bergamottöl | 50 | 50 |
| Labdanumöl (franz.) | 30 | 30 |
| Eichenmoos-Extrakt | 12 | 12 |
| Citronellol | 18 | 18 |
| Cumarin | 13 | 13 |
| Keton-Moschus | 12 | 12 |
| Nerol | 10 | 10 |
| Indol, 10% Lsg. | 3 | 3 |
| Linalool | 45 | 45 |
| Terpinylacetat | 95 | 95 |
| 2-Ethyl-2-prenyl-3-hexenol | – | 62 |
|  | 938 | 1000 |

3

Die Komposition a besitzt einen frischen, herb-krautigen Duft mit balsamischen Nebennoten. Durch Zugabe von 62 Gewichtsteilen der erfindungsgemäßen Verbindung (gemäß b) wird die krautige, leicht campfrige Note weicher. Die balsamischen Nebennoten werden betont.

Beispiel 4

Stabilitätstest

2-Ethyl-2-prenyl-3-hexenol wurde in verschiedenen Medien 60 Tage beiI 40° C aufbewahrt. Danach wurde auf Farb- und Geruchsveränderungen geprüft und mit frisch bereiteten Lösungen verglichen. Die Lösungen wurden ferner mittels Dünnschichtchromatographie untersucht.

Es wurden bei jeweils vier Wiederholungen 0,5 gew.-%ige Lösungen von 2-Ethyl-2-prenyl-3-hexenol in 60 gew.-%igem, wässrigem Ethanol bereitet und mit 0,1 n HCl bzw. 0,1 n NaOH auf den gewünschten pH-Wert gebracht.

Testlösung 1 pH = 2
Testlösung 2 pH = 7
Testlösung 3 pH = 13

Unter den oben beschriebenen Bedingungen blieb 2-Ethyl-2-prenyl-3-hexenol in allen Testlösungen stabil : Es konnten keine Farb- oder Geruchsveränderungen festgestellt werden. Die Dünnschichtchromatogramme gaben keinen Hinweis auf chemische Veränderungen.

Vergleichsbeispiel 1

Die Testmethode gemäß Beispiel 4 wurde wiederholt mit der Abänderung, daß anstatt 2-Ethyl-2-prenyl-3-hexenol der gemäß Stand der Technik bekannte Riechstoff 2-Ethyl-2-prenyl-3- hexenal untersucht wurde.

Ergebnis

Die Vergleichssubstanz ist bei pH = 7 stabil. In alkalischer Lösung (pH = 13) waren bereits nach einem Tag, in saurer Lösung (pH = 2) nach 3 Tagen gelb-braune Verfärbungen eingetreten. Das Geruchsbild war deutlich verändert. Im Dunn-schichtchromatogramm wurden im Vergleich zu einer frisch bereiteten, unbehandelten Probe Fremdsubstanzen nachgewiesen.

**Patentansprüche**

1. 2-Ethyl-2-prenyl-3-hexenol.

2. Verfahren zur Herstellung der Verbindung nach Anspruch 1, dadurch gekennzeichnet, daß 2-Ethyl-2-prenyl-3-hexenal in Gegenwart von Aluminium-isopropylat reduziert wird.

3. Verwendung der Verbindung nach Anspruch 1 als Riechstoff oder als Bestandteil von Riechstoffmischungen.

**Claims**

1. 2-Ethyl-2-prenyl-3-hexenol.

2. Process for the production of the compound according to claim 1, characterized in that 2-ethyl-2-prenyl-3-hexenol is reduced in the presence of aluminium isopropylate.

3. Use of the compound according to claim 1 as fragrant substance or as a component of mixtures of fragrant substances.

**Revendications**

1. Ethyl-prényl-2 hexène-3 ol.

2. Procédé de préparation du composé selon la revendication 1, procédé caractérisé en ce qu'on réduit l'éthyl-2 prényl-2 hexène-3 al en présence d'isopropylate d'aluminium.

3. Application du composé selon la revendication 1 comme parfum ou comme constituant de mélanges parfums.